# EUROPEAN PATENT SPECIFICATION

(11) **EP 2 694 004 B1**
(45) Date of publication and mention of the grant of the patent: **10.06.2015**
(21) Application number: 12715579.4
(22) Date of filing: 03.04.2012
(51) Int. Cl.: A61F 13/02, A61L 15/58, A61L 24/00

(54) **AN ADHESIVE PATCH**
KLEBEPFLASTER
PIÈCE ADHÉSIVE

(30) Priority: 04.04.2011 DK 201170157
(43) Date of publication of application: 12.02.2014
(73) Proprietor: Coloplast A/S, 3050 Humlebæk (DK)
(72) Inventor: SIDENIUS, Martin, DK-2100 Copenhagen (DK); MARCUSSEN, Jan, DK-2630 Taastrup (DK); BONNÉ, Tune Bjarke, DK-3480 Fredensborg (DK)
(86) International application number: PCT/DK2012/050105
(87) International publication number: WO 2012/136218

(56) References cited:
- EP-A1- 1 527 789
- WO-A1-98/53771
- WO-A2-2008/063623
- GB-A- 2 089 351
- US-A- 5 714 225

## Description

### Background of the Invention

The invention relates to an adhesive patch suitable for application to the skin or mucosa. The objective of this invention is to develop a patch for cold sore that can offer the properties of the adhesive patch in conjunction with the anti-viral and wound healing properties of zinc.

Adhesive dressings or patches are often used for covering and treating different skin conditions, such as scratches, wounds, acne, eczema, herpes and warts and may both serve to camouflage irregularities and treat the skin condition.

Hydrocolloid dressings or patches provide moist wound healing and may in itself facilitate healing of the skin condition. However, the healing effect may further be enhanced by the presence of various active ingredients.

Zinc is being used in a lot of different products, and, for more than 75 years, it has been used in eye drops as mild astringent for relief of eye irritation. Furthermore, zinc is also used in infusion solutions for zinc deficiency, and, in general, zinc is being used for eczema and in skin protecting lotions and gaze.

Zinc accumulates into virion membrane in the glycoprotein-dependent components and inhibits the function of glycol proteins. The inactivated virus is not able to penetrate and, to some extent, adsorb into human cells. Approximately 300 enzymes need zinc for proper functioning. Many of these zinc-dependent processes are required for wound healing, such as collagen synthesis and cell division. Consequently, zinc is an essential nutrient for normal wound healing.

The zinc compound used for creams and ointments are usually in the form of zinc oxide. However, zinc oxide is white and thus clearly visible and not suitable for a discrete patch. Moreover, the release of zinc from zinc oxide is low.

### Detailed Description of the Present Invention

The invention relates to a patch for covering a portion of the anatomical surface of a living being, said patch being able to adhere to the skin or mucosa, and/or a wound, said patch comprising a backing layer and a layer of a skin-friendly adhesive for adhering to the skin or mucosa, wherein said adhesive comprises hydrocolloid particles and zinc sulphate particles, wherein the ratio between the hydrocolloid particles and the zinc sulphate particles is from 4 to 22 w/w.

Zinc sulphate has been chosen as the medicinal substance, due to the fact that there is clinical evidence from literature that zinc sulphate has a beneficial effect towards herpes simplex and because it is possible to develop a patch with zinc sulphate that is transparent.

A certain release of zinc is needed in order to obtain an anti viral effect. However, too high levels of released zinc may be cytotoxic and is highly undesired. Hence, the release of zinc should be high enough to facilitate an anti viral effect, but low enough to avoid a cytotoxic reaction.

The concentration of zinc has an impact on the anti-viral activity of a patch containing zinc; however, it has surprisingly been shown that the amount of hydrocolloids has an effect on the release of zinc from the patch and therefore the anti-viral activity. High hydrocolloid content yields high release and therefore better anti viral effect.

However, if the adhesive comprises a high amount of hydrocolloid, the retention of moisture in the hydrocolloid may cause changes in the adhesive, such as swelling, loss of cohesion and loss of adhesive tack of the patch and thereby decrease the wear time of the patch. On the other hand, a low amount of hydrocolloids may result in a poor release of zinc as well as it may impart wet tack and moisture absorption properties.

Thus, in order to achieve an adhesive patch with an anti viral effect and long wear time, the amounts of hydrocolloid and zinc in the adhesive should be carefully balanced facilitating a high anti-viral activity without the patch being cytotoxic.

The ratio between the hydrocolloid particles and the zinc sulphate particles may be from 4 to 20 w/w, 4.5-15 w/w, 4.5-10 w/w or 4.5-7 w/w. In one embodiment, the ratio between the hydrocolloid particles and the zinc sulphate particles is 5 w/w.

The adhesive of the patch may comprise a total amount of particles of 20-35, 20-34, 20-30, 20-27 or 22-25 % w/w. In a preferred embodiment, the amount of particles is 23 %. Here, by particles is meant hydrocolloid particles and zinc sulphate which are typically both added to the adhesive in the form of a powder.

Zinc may be achieved in various forms, most commonly used is zinc oxide. However, zinc oxide is white and the adhesive is turning white and non-translucent when zinc oxide is added, thereby turning the patch rather visible and non-discrete. Furthermore, the release of zinc oxide from the patch is poor.

Zinc sulphate compounds are very suitable for incorporation in a patch, for example, in the form of zinc sulphate monohydrate or zinc sulphate heptahydrate. Both the monohydrate and the heptahydrate show sufficient release and anti viral effect and do not affect the appearance of the patch. A transparent or translucent adhesive may be obtained.

In a preferred embodiment of the invention, the zinc sulphate is in the form of zinc sulphate monohydrate. The monohydrate has a particle size facilitating easy incorporation into the adhesive and is advantageous especially when coating the adhesive into thin layers.

A low particle size of the zinc sulphate compound facilitates coating the adhesive into very thin layers whereas a high particle size may provide a higher release. In one embodiment of the invention, the zinc sulphate compound is in the form of a micronized powder.

In one embodiment, the zinc sulphate may be in the form of zinc sulphate heptahydrate.

The zinc particles may be present in an amount of 2-4.5, such as 3-4 or such as 3.2-4.0 % w/w.

The thickness of the adhesive layer of the patch may be in the range of 20-300 µm, such as 30-200 µm, such as 25-150 µm, such as 30-100 µm or such as 50-80 µm.

It has been found that a patch with such thickness and vapour permeability provides a non-occlusive adhesive patch, i.e. one that enables moisture on, e.g., a skin surface to evaporate through the dressing sheet, so as to prevent undesired accumulation of moisture which could cause the dressing sheet to lose its adhering contact to the skin or promote bacterial growth between the sheet and the skin.

Moreover, the low thickness of the dressing sheet results in a discrete appearance once applied to the application site. The dressing or patch is especially suitable for use in the face or other visible or exposed areas of the body where it is desired that the patch blends into the skin and appears almost invisible.

The presence of hydrocolloid in the adhesive provides a good environment for moist wound healing as well as for other skin conditions. By incorporating hydrocolloid in the adhesive, the patch is able to handle moisture in most conditions and the release of zinc is enhanced.

The present invention discloses a patch that provides optimal conditions for moist wound healing and long wear time when applied to the skin or mucosa (e.g. lips).

A patch according to the invention may be used for application to mucosa.

The patch of the present invention may have a wear time on skin of at least 12 hours, more preferred at least 24 hours, even more preferred at least 36 hours and most preferred at least 48 hours. When applied to the mucosa and other places where the patch is exposed to high humidity and friction, such as in the lip region, the wear time may naturally be shorter. For application to the lip region, the wear time is preferably at least 2 hours, more preferred at least 3 hours and most preferred at least 4 hours. The wear time may be at least 8-12 hours.

A patch according to the invention may be used for application to the lip.

By application to the lip or the lip region is meant that the whole area of the patch can be applied to the lip or a part of the patch can be applied to the lip and the remaining part applied to the surrounding skin. Especially, the patch can be applied to the edge of the lip and the major part of the patch is applied to the skin.

It is difficult to apply an adhesive dressing or patch to the lip region, partly due to the high mobility and stretching, and partly due to the humid environment. Part of the lip is "dry" skin and part of it is "wet" skin or mucosa. The dry part is often exposed to moisture, e.g. during eating, drinking or simply licking the lips. A dressing or patch capable of sticking to a wet surface, such as mucosa, an exuding wound and/or skin exposed to moisture (the dry part of the lip) may preferably be provided with an adhesive having wet tack and the backing layer is preferably water impervious, but vapour permeable.

A patch according to the invention may be used for facial application.

The thickness of the adhesive layer of the patch of the present invention may be substantially constant over the surface or the patch may have a thicker portion at the centre of the patch, surrounded by a thinner periphery, i.e. a bevelled edge. It has surprisingly been shown that a better performance for the patch is achieved by having a thin edge portion. The thin periphery of the patch decreases the risk of rolling-up of the edge portion. The rolling up of the edge portion may lead to reduced wear time and is undesired. Furthermore, the thin edge portion is less exposed to water coming from outside, and renders it possible to obtain an extreme water-block.

The surface area of the dressing sheet may, e.g., be 5-25 cm², such as 10-20 cm², or smaller, such as less than 5 cm², such as at most 4 cm², such as at most 2 cm², such as in the range of 1-2 cm², or smaller, such as 0.08 - 1 cm², such as 0.1 - 0.8 cm² or such as 0.12 - 0.5 cm². For facial application, e.g. of a thin film patch, the surface area is usually less than 5 cm².

The adhesive of the patch of the invention may be any suitable skin-friendly adhesive. The adhesive further comprises particles of hydrocolloids and/or super absorbing particles or fibres.

The skin-friendly adhesive may be any skin-friendly adhesive known per se for production of medical articles, which are to be adhered to human skin. The adhesive may suitably be of the type disclosed in US patent Nos. 4,231,369, 4,367,732, 4,867,748, and 5,714,225. Especially preferred are the adhesives disclosed in US patent Nos. 4,367,732 and 5,714,225.

In one embodiment of the invention, the patch of the present invention may be in the form of a mono-phase adhesive, i.e. made from one adhesive component, or, in accordance with another embodiment of the invention, it may be in the form of a two-zone adhesive, e.g. of the general type disclosed in US patent No. 5,714,225, i.e. a part of or all of the adhesive areas of the patch having a maximum thickness being constituted by more than one type of adhesive.

The particle size of the hydrocolloid particles may influence the thickness of the adhesive layer, as it is difficult to prepare an adhesive layer being thinner than the size of the hydrocolloid particles.

The physical form of conventional hydrocolloids is relative coarse and irregular particles, typically about 60-300 µm, and the particles are in the form of a dry powder. In order to obtain finer particles, the hydrocolloid may be milled and/or sifted.

It is preferred that the amount of hydrocolloid particles is 15-33, more preferred 15-30 even more preferred 15-25 and most preferred 18-23 % w/w.

Suitable hydrocolloids for the patch of the present invention include synthetic polymers prepared from single or multiple monomers, naturally occurring hydrophilic polymers or chemically modified naturally occurring hydrophilic polymers. The hydrocolloid polymers may be linear or cross-linked. This include natural or chemically modified natural polymers like cellulosics such as CMC, chitosan, pectin, guar gum, starches or dextrines, collagenes and gelatine and synthetic polymers like polyacrylic acid, polyvinylalcohol/acetate, polyhydroxyalkyl acrylates and methacrylates, polyacrylamides, polystyrene sulfonates, polyvinyl pyrilidone, polyglycols, copolymers, grafts of such, copolymers or compositions of such.

In a preferred embodiment of the invention, the hydrocolloid particles have an average size being substantially less than 125 µm, more preferred less than 100 µm, even more preferred less than 75 µm and most preferred less than 50 µm.

The patch of the invention is suitable for covering cuts, graces and abrasions, scars, wrinkles, discolouring of the skin or the like. The patch may be especially suitable for treatment of herpes simplex and herpes labialis. The patch may be applied to the lip or lip region being discrete and without causing discomfort.

It is suitable that the backing layer is a substantially water impervious film which protects the adhesive from being adversely affected when the wearer is bathing or in case of incidental wetting of the area and especially when the adhesive is water absorbing.

The backing layer may be any water impervious layer or film and may be of any suitable material known per se for use in the preparation of wound dressings e.g. a foam, a nonwoven layer or a polyurethane, polyethylene, polyester or polyamide film. In accordance with the invention, it has been found in practice that the use of a thinner backing layer or film than is normally used when preparing medical dressings, an improved stretchability and adaptability is obtained at the same time as the modulus is reduced.

An especially suitable material for use as a water impervious film is a polyurethane film. A preferred low friction film material is disclosed in US patent No. 5,643,187.

According to an embodiment of the invention, the backing layer may be a vapour or moisture permeable, but liquid impermeable layer or film. In this case the patch of the invention may provide a good moisture transmission rate and is able to transport a large quantity of moisture through the patch and away from the skin. Both the chemical composition and physical construction of the adhesive layer, and the chemical and physical construction of the backing layer affect the water vapour permeability. With regard to the physical construction, the backing layer may be continuous (no holes, perforations, indentations, no added particles or fibres affecting the water vapour permeability) or discontinuous (it has holes, perforations, indentations, added particles or fibres affecting the water vapour permeability).

The backing layer may have a suitable thickness for the intended use. If the patch were desired for an "invisible" face patch, a rather thin film would be appropriate. It is preferred that the backing layer has a thickness of less than 30 µm.

A preferred thickness of the backing layer may be below 20 microns, more preferred about 12-18 microns, e.g. about 15 microns, thus resulting in a significant decrease of the modulus, compared to a film that is normally used when preparing medical dressings. An improved stretchability and adaptability is obtained at the same time as the modulus is reduced.

### Description of the Experimental

### EXAMPLE 1

An adhesive matrix was heated and the zinc sulphate and hydrocolloid particles were added to the matrix. The mixing process was continued until the powders were homogenously distributed in the mass. The adhesive mixture was coated onto a 15 micron polyurethane film in an adhesive thickness of 55-95 micron.

The following samples were prepared:

| | % Hydrocolloid | % ZnSO4, H2O |
|---|---|---|
| A | 19,2 | 3,8 |
| B | 30 | 1,5 |
| C | 26 | 4 |

## Claims

1. A patch for covering a portion of the anatomical surface of a living being, said patch being able to adhere to the skin or mucosa, and/or a wound, said patch comprising a backing layer and a layer of a skin-friendly adhesive for adhering to the skin or mucosa, **characterized in that** said adhesive comprises hydrocolloid particles and zinc sulphate particles wherein the ratio between the hydrocolloid particles and the zinc sulphate particles is from 4 to 22 w/w.

2. The patch according to claim 1, wherein the ratio between the hydrocolloid particles and the zinc sulphate particles is from 4.5 - 10 w/w.

3. The patch according to any of the preceding claims, wherein the ratio between the hydrocolloid particles and the zinc sulphate particles is from 4.5 to 7 w/w.

4. The patch according to any of the preceding claims, wherein the adhesive comprises 20-35% w/w particles.

5. The patch according to any of the preceding claims, wherein the zinc sulphate is micronized.

6. The patch according to any of the preceding claims, wherein the zinc sulphate is zinc sulphate monohydrate.

7. The patch according to any of claims 1-5, wherein the zinc sulphate is zinc sulphate heptahydrate.

8. The patch according to any of the preceding claims, wherein the particle size of the hydrocolloid is less than 100 µm.

9. The patch according to any of the preceding claims, wherein the hydrocolloid is CMC.

10. The patch according to any of the preceding claims, wherein the thickness of the patch is from 20 to 300 µm.

## Patentansprüche

1. Pflaster zum Abdecken eines Teils der anatomischen Oberfläche eines Lebewesens, wobei das Pflaster auf der Haut oder Schleimhaut und/oder einer Wunde haften kann, wobei das Pflaster eine Rückschicht und eine Schicht aus einem hautfreundlichen Klebstoff zum Aufkleben auf die Haut oder Schleimhaut umfasst, **dadurch gekennzeichnet, dass** der Klebstoff Hydrokolloid-Teilchen und Zinksulfat-Teilchen umfasst, wobei das Gewichtsverhältnis zwischen den Hydrokolloid-Teilchen und den Zinksulfat-Teilchen 4 bis 22 beträgt.

2. Pflaster nach Anspruch 1, wobei das Gewichtsverhältnis zwischen den Hydrokolloid-Teilchen und den Zinksulfat-Teilchen 4,5-10 beträgt.

3. Pflaster nach einem der vorhergehenden Ansprüche, wobei das Gewichtsverhältnis zwischen den Hydrokolloid-Teilchen und den Zinksulfat-Teilchen 4,5 bis 7 beträgt.

4. Pflaster nach einem der vorhergehenden Ansprüche, wobei der Klebstoff 20-35 Gew.-% Teilchen umfasst.

5. Pflaster nach einem der vorhergehenden Ansprüche, wobei das Zinksulfat mikronisiert ist.

6. Pflaster nach einem der vorhergehenden Ansprüche, wobei es sich bei dem Zinksulfat um Zinksulfatmonohydrat handelt.

7. Pflaster nach einem der Ansprüche 1-5, wobei es sich bei dem Zinksulfat um Zinksulfatheptahydrat handelt.

8. Pflaster nach einem der vorhergehenden Ansprüche, wobei die Teilchengröße des Hydrokolloids weniger als 100 µm beträgt.

9. Pflaster nach einem der vorhergehenden Ansprüche, wobei es sich bei dem Hydrokolloid um CMC handelt.

10. Pflaster nach einem der vorhergehenden Ansprüche, wobei die Dicke des Pflasters 20 bis 300 µm beträgt.

## Revendications

1. Pièce pour couvrir une partie de la surface anatomique d'un être vivant, ladite pièce étant capable d'adhérer à la peau ou à une muqueuse, et/ou une plaie, ladite pièce comprenant une couche de dossier et une couche d'un adhésif hypoallergénique à coller à la peau ou à la muqueuse, **caractérisée en ce que** ledit adhésif comprend des particules d'hydrocolloïdes et des particules de sulfate de zinc, dans laquelle le rapport de poids entre les particules d'hydrocolloïdes et les particules de sulfate de zinc est compris entre 4 et 22.

2. Pièce selon la revendication 1, dans laquelle le rapport de poids entre les particules d'hydrocolloïdes et les particules de sulfate de zinc est compris entre 4,5 et 10.

3. Pièce selon l'une quelconque des revendications précédentes, dans laquelle le rapport de poids entre les particules d'hydrocolloïdes et les particules de sulfate de zinc est compris entre 4,5 et 7.

4. Pièce selon l'une quelconque des revendications précédentes, dans laquelle l'adhésif comprend entre 20 % et 35 % poids/poids de particules.

5. Pièce selon l'une quelconque des revendications précédentes, dans laquelle le sulfate de zinc est micronisé.

6. Pièce selon l'une quelconque des revendications précédentes, dans laquelle le sulfate de zinc est le monohydrate de sulfate de zinc.

7. Pièce selon l'une quelconque des revendications 1 à 5, dans laquelle le sulfate de zinc est l'heptahydrate de sulfate de zinc.

8. Pièce selon l'une quelconque des revendications précédentes, dans laquelle la taille de particule de l'hydrocolloïde est inférieure à 100 µm.

9. Pièce selon l'une quelconque des revendications précédentes, dans laquelle l'hydrocolloïde est le CMC.

10. Pièce selon l'une quelconque des revendications précédentes, dans laquelle l'épaisseur de la pièce est comprise entre 20 µm et 300 µm.
